# EUROPEAN PATENT SPECIFICATION

(11) **EP 1 461 083 B1**
(45) Date of publication and mention of the grant of the patent: **29.03.2006**
(21) Application number: 02787763.8
(22) Date of filing: 18.12.2002
(51) Int. Cl.: A61K 45/06, A61K 31/40, A61K 31/415, A61P 35/00

(54) **COMBINED THERAPY AGAINST TUMORS COMPRISING SUBSTITUTED ACRYLOYL DISTAMYCIN DERIVATIVES AND PROTEIN KINASE (SERINE/THREONINE KINASE) INHIBITORS**
KOMBINIERTE TUMORTHERAPIE AUF DER BASIS VON SUBSTITUIERTEN ACRYLOYL-DISTAMYCIN-DERIVATEN UND PROTEIN-KINASE (SERIN/THREONIN-KINASE) INHIBITOREN
THERAPIE COMBINEE CONTRE LES TUMEURS, REPOSANT SUR L'UTILISATION DE DERIVES DE DISTAMYCINE D'ACRYLOYLE SUBSTITUES ET D'INHIBITEURS DE PROTEINE KINASE (SERINE/THREONINE KINASE)

(30) Priority: 02.01.2002 EP 02075052
(43) Date of publication of application: 29.09.2004
(73) Proprietor: Pharmacia Italia S.p.A., 20152 Milano (IT)
(72) Inventor: GERONI, Maria, Cristina, I-20149 Milan (IT); FOWST, Camilla, I-20153 Milan (IT); COZZI, Paolo, I-20133 Milan (IT)
(74) Representative: Modiano, Micaela Nadia
(86) International application number: PCT/EP2002/013092
(87) International publication number: WO 2003/055522

(56) References cited:
- WO-A-01/97789
- WO-A-01/97790
- M.CIOMEI E.A.: "Decreased tyrosine phosphorylation in tumour cells resistant to FCE 24517 (tallimustine)" BRITISH JOURNAL OF CANCER, vol. 72, no. 6, 1995, pages 1504-1508, XP008015252
- S.MARCHINI E.A.: "Alpha-bromoacryloyl derivative of distamycin A (PNU 151807):a new non-covalent minor groove DNA binder with antineoplastic activity" BRITISH JOURNAL OF CANCER, vol. 80, no. 7, 1999, pages 991-997, XP008015251

## Description

The present invention relates to the field of cancer treatment and provides an antitumor composition comprising a substituted acryloyl distamycin derivative, more particularly an α-bromo- or α-chloro-acryloyl distamycin derivative, and a protein kinase (serine/threonine and tyrosine kinases) inhibitor, having a synergistic antineoplastic effect.

Distamycin A and analogues thereof, hereinafter referred to as distamycin and distamycin-like derivatives, are known in the art as cytotoxic agents useful in antitumor therapy.
Distamycin A is an antibiotic substance with antiviral and antiprotozoal activity, having a polypyrrole framework [*Nature* 203: 1064 (1964); *J. Med. Chem.* 32: 774-778 (1989)]. The international patent applications WO 90/11277, WO 98/04524, WO 98/21202, WO 99/50265, WO 99/50266 and WO 01/40181, disclose acryloyl distamycin derivatives wherein the amidino moiety of distamycin is optionally replaced by nitrogen-containing ending groups such as, for instance, cyanamidino, N-methylamidino, guanidino, carbamoyl, amidoxime, cyano and the like, and/or wherein the polypyrrole framework of distamycin, or part of it, is replaced by varying carbocyclic or heterocyclic moieties.
The present invention provides, in a first aspect, a pharmaceutical composition for use in antineoplastic therapy in mammals, including humans, comprising a pharmaceutically acceptable carrier or excipient;
- an acryloyl distamycin derivative of formula (I): 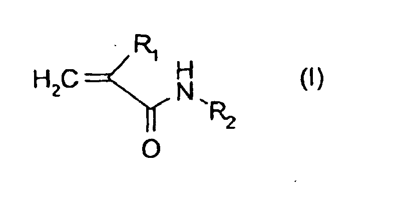 wherein:
   R₁ is a bromine or chlorine atom;
   R₂ is a distamycin or a group of formula (II) as defined in claim 1, or a pharmaceutically acceptable salt thereof; and
- a protein kinase inhibitor.

The present invention includes, within its scope, the pharmaceutical compositions comprising any of the possible isomers covered by the compounds of formula (I), both considered separately or in admixture, as well as the metabolites and the pharmaceutically acceptable bio-precursors (otherwise known as pro-drugs) of the compounds of formula (I).

In the present description, unless otherwise specified, with the term distamycin or R₂ we intend any moiety of formula (II) structurally closely related to distamycin itself, for instance by optionally replacing the ending amidino moiety of distamycin and/or its polypyrrole framework, or part of it, for instance as set forth below.

Protein kinases, hereinafter shrortly referred to as PKs, are a large family of homologous proteins [see, for a reference, *J. Clin. Invest.* 105: 3 (2000); *Cancer Chemotherapy and Biological Response Modifiers, Annual 19* Chapter 11, 236 (2001)].

PKs, as components of signal transduction pathways, play a central role in diverse biological processes such as control of cell growth, metabolism, differentiation, and apoptosis. The development of selective PK inhibitors that can block or modulate diseases with defects in these signaling pathways, has been considered as a promising approach for the development of new anticancer drugs. A selection of these agents is shown in Table 1.

**Table 1: Low Molecular weight ATP-competitive protein kinase inhibitors in clinical development**

| **Target Kinase** | **Name** |
|---|---|
| Bcr-Abl | STI571 (Gleevec; Imatinib) |
| EGF-R | ZD-1839 (Iressa) |
| | OSI-774 (Tarceva) |
| | PKI166 |
| | EKB-569 |
| | GW572016 |
| PKC/Trk | CEP 2563 |
| PKC | UCN-01 |
| | GCP41251(STI412) |
| | Safingol |
| | Perifosine |
| VEGF-R | SU 5416 (Semaxanib) |
| | CGP 79787 |
| | CP-564959 |
| | ZD 6474 |
| | ZD 2171 |
| | SU-11248 |
| CDKs | Flavopiridol |
| | CI-202 |

The compositions of the invention may be thus comprised by the aforementioned acryloyl distamycin derivative of formula (I) and a protein kinase inhibitor, as listed in table 1.
According to a preferred embodiment of the invention, the PKs inhibitor is selected from STI571 (Gleevec; Imatinib - inhibitor of Bcr-Abl tyrosine kinase), ZD-1839 (Iressa - inhibitor of epidermal growth factor receptor 1 tyrosine kinase), OSI-774 (Tarceva - inhibitor of epidermal growth factor receptor 1 tyrosine kinase) and SU 5416 (Semaxanib - tyrosine kinase inhibitor that inhibits three distinct growth factor receptor targets).

According to another preferred embodiment of the invention, herewith provided are the above pharmaceutical compositions wherein, within the acryloyl distamycin derivative of formula (I), R₁ has the above reported meanings and R₂ is a group of formula (II) below: 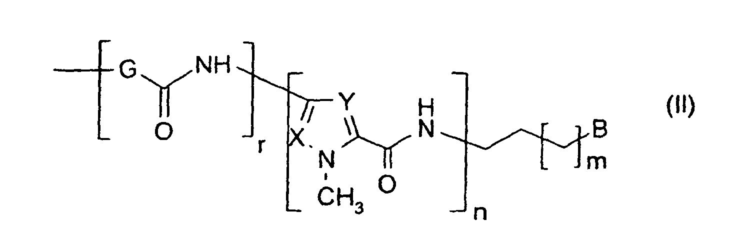 wherein
m is an integer from 0 to 2;
n is an integer from 2 to 5;
r is 0 or 1;
X and Y are, the same or different and independently for each heterocyclic ring, a nitrogen atom or a CH group;
G is phenylene, a 5 or 6 membered saturated or unsaturated heterocyclic ring with from 1 to 3 heteroatoms selected among N, O or S, or it is a group of formula (III) below: 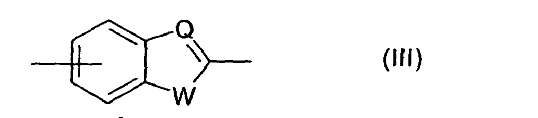 wherein Q is a nitrogen atom or a CH group and W is an oxygen or sulfur atom or it is a group NR₃ wherein R₃ is hydrogen or C₁-C₄ alkyl;
B is selected from the group consisting of 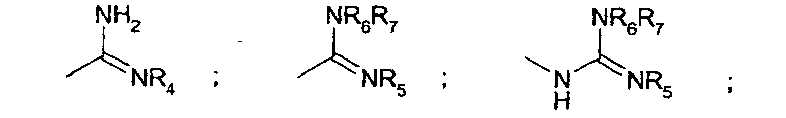 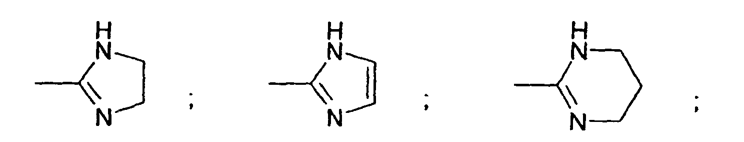 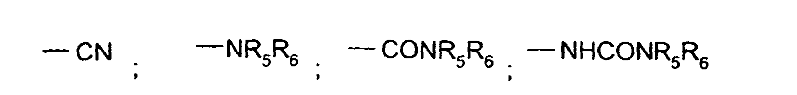 wherein R₄ is cyano, amino, hydroxy or C₁-C₄ alkoxy; R₅, R₆ and R₇, the same or different, are hydrogen or C₁-C₄ alkyl.

In the present description, unless otherwise specified, with the term C₁-C₄ alkyl or alkoxy group we intend a straight or branched group selected from methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy or tert-butoxy.
Preferably, the pharmaceutical compositions of the invention comprise the above acryloyl distamycin derivative of formula (I) wherein R₁ is bromine or chlorine; R₂ is the above group of formula (II) wherein r is 0, m is 0 or 1, n is 4 and B has the above reported meanings.
Still more preferred, within this class, are the pharmaceutical compositions comprising the compounds of formula (I) wherein R₁ is bromine or chlorine; R₂ is the above group of formula (II) wherein r is 0, m is 0 or 1, n is 4, X and Y are both CH groups and B is selected from:

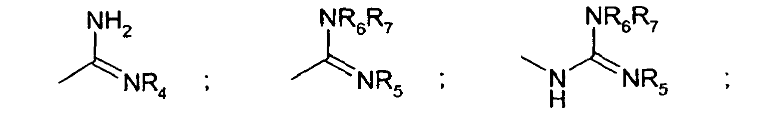

-CN ; -CONR₅R₆ ; -NHCONR₅R₆

wherein R₄ is cyano or hydroxy and R₅, R₆ and R₇, the same or different, are hydrogen or C₁--C₄ alkyl.

Even more preferred compositions of the invention are those comprising a compound of formula (I) wherein R₁ is bromine, R₂ is the above group of formula (II) wherein r and m are 0, n is 4, X and Y are CH, B is a group of formula: 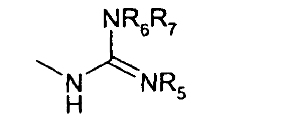 wherein R₅, R₆ and R₇ are hydrogen atoms, optionally in the form of a pharmaceutically acceptable salt thereof.
Pharmaceutically acceptable salts of the compounds of formula (I) are those with pharmaceutically acceptable inorganic or organic acids such as, for instance, hydrochloric, hydrobromic, sulfuric, nitric, acetic, propionic, succinic, malonic, citric, tartaric, methanesulfonic, p-toluenesulfonic acid and the like.

Examples of preferred acryloyl distamycin derivatives of formula (I), within the compositions object of the invention, for instance in the form of pharmaceutically acceptable salts, preferably with hydrochloric acid, are:
1. N-[5-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino]-1-methyl-1H-pyrrol-2-yl[carbonyl]amino]-1-methyl-1H-pyrrole-2-carboxamide hydrochloride (Brostallicin);
2. N-(5-{[(5-{[(5-{[(2-{[amino(imino)methyl]amino}propyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride;
3. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride;
4. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-imidazole-2-carboxamide hydrochloride;
5. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromoacryloyl)amino]-1-methyl-1H-pyrazole-5-carboxamide hydrochloride;
6. N-(5-{[(5-{[(5-{[(3-amino-3-oxopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromoacryloyl)amino]-1-methyl-1H-pyrazole-5-carboxamide;
7. N-(5-{[(5-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-chloroacryloyl)amino]-1-methyl-1H-pyrrole-2-carboxamide hydrochloride;
8. N-(5-{[(5-{[(3-{[amino(imino)methyl]amino}propyl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride;
9. N-(5-{[(5-{[(3-amino-3-iminopropyl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride; and
10. N- {5-[({5-[({5-[({3-[(aminocarbonyl)amino]propyl}amino)carbonyl]-1-methyl-1 H-pyrrol-3-yl}amino)carbonyl]-1-methyl-1H-pyrrol-3-yl}amino)carbonyl]- I - methyl-1H-pyrrol-3-yl}-4-[(2-bromoacryloyl)amino]-1-methyl-1H-pyrrole-2-carboxamide.

The above compounds of formula (I), either specifically identified as such or by means of the general formula, are known or easily prepared according to known methods as reported, for instance, in the aforementioned international patent applications WO 90/11277, WO 98/04524, WO 98/21202, WO 99/50265 and WO 99/50266 as well as in WO 01/40181.

The present invention further provides a product, otherwise referred to as kit of parts, comprising an acryloyl distamycin derivative of formula (I), as defined above, and a PK inhibitor, as a combined preparation for simultaneous, separate or sequential use in antitumor therapy.
A further aspect of the present invention is the treatment of a mammal, including humans, suffering from a neoplastic disease state, which comprises administering to said mammal the above acryloyl distamycin derivative of formula (I) and a PK inhibitor, in amounts effective to produce a synergistic antineoplastic effect. The present invention also provides a use for lowering the side effects caused by antineoplastic therapy with an antineoplastic agent in a mammal in need thereof, including humans, comprising administering to said mammal a combined preparation comprising a PK inhibitor and an acryloyl distamycin derivative of formula (I), as defined above, in amounts effective to produce a synergistic antineoplastic effect.

By the term "synergistic antineoplastic effect", as used herein, it is meant the inhibition of the growth tumor, preferably the complete regression of the tumor, by administering an effective amount of the combination comprising an acryloyl distamycin derivative of formula (I) and a PK inhibitor to mammals, including humans.
By the term "administered " or "administering", as used herein, it is meant parenteral and/or oral administration; the term "parenteral" means intravenous, subcutaneous and intramuscular administration.
In the use of the present invention, the acryloyl distamycin derivative may be administered simultaneously with the PK inhibitor or, alternatively, both compounds may be administered sequentially in either order.
In this respect, it will be appreciated that the actual preferred use and order of administration will vary according to, inter alia, the particular formulation of the acryloyl distamycin of formula (I) being used, the particular formulation of the PK inhibitor being used, the particular tumor model being treated as well as the particular host being treated.
To administer the acryloyl distamycin derivative of formula (I), according to the use of the invention, the course of therapy generally employed comprises doses varying from about 0.05 to about 100 mg/m² of body surface area and, more preferably, from about 0.1 to about 50 mg/m² of body surface area.
For the administration of the PK inhibitor, according to the use of the invention, the course of therapy generally employed may be as follows.
For the administration of STI571 (Imatinib), doses varying from about 5 mg/day to about 5000 mg/day and, more preferably, from about 30 to about 1000 mg/day.
For the administration of ZD 1839 (Iressa) doses varying from about 5 mg/day to about 10000 mg/day and, more preferably, from about 50 to about 1000 mg/day.
For the administration of OSI-774 (Tarceva) doses varying from about 5 mg/day to about 10000 mg/day and, more preferably, from about 50 to about 1000 mg/day.
For the administration of SU 5416 (Semaxanib) doses varying from about 1 mg/m² to about 1000 mg/m² of body surface area and, more preferably, from about 10 to about 500 mg/m² of body surface area.

The antineoplastic therapy of the present invention is particularly suitable for treating breast, ovary, lung, colon, kidney, stomach, pancreas, liver, melanoma, leukemia and brain tumors in mammals, including humans.
In a further aspect, the present invention is directed to a pharmaceutical composition comprising an effective amount of an acryloyl distamycin derivative of formula (I), as defined above, and a PK inhibitor, in the preparation of a medicament for use in the prevention or treatment of metastasis or in the treatment of tumors by inhibition of angiogenesis.
As the effect of an acryloyl distamycin derivative of formula (I) and a PK inhibitor is significantly increased without a parallel increase of toxicity, the combined therapy of the present invention enhances the antitumoral effects of the acryloyl distamycin derivative and of the PK inhibitor and, hence, provides the most effective and least toxic treatment for tumors.

## Claims

1. A pharmaceutical composition comprising a pharmaceutically acceptable carrier or excipient and, as active ingredient;
- an acryloyl distamycin derivative of formula (I): 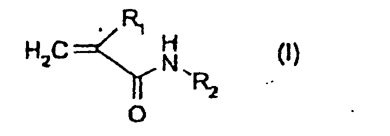 wherein:
R₁ is a bromine or chlorine atom;
R₂ is a distamycin or a group of formula (II) 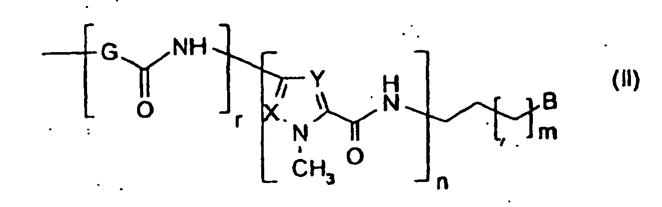 wherein
m is an integer from 0 to 2;
n is an integer from 2 to 5;
r is 0 or 1;
X and Y are, the same or different and independently for each heterocyclic ring, a nitrogen atom or a CH group;
G is phenylene, a 5 or 6 membered saturated or unsaturated heterocyclic ring with from 1 to 3 heteroatoms selected among N, O or S, or it is a group of formula (III) below: 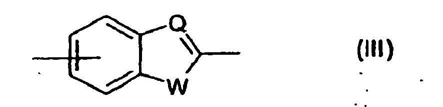 wherein Q is a nitrogen atom or a CH group and W is an oxygen or sulfur atom or it is a group NR₃ wherein R₃ is hydrogen or C₁-C₄ alkyl;
B is selected from the group consisting of 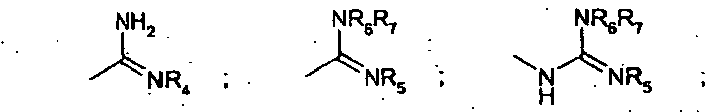 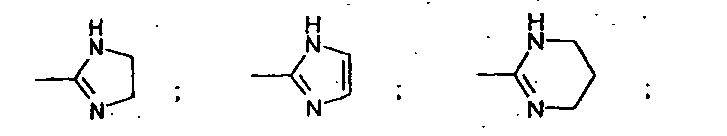
―CN ―NR₅R₆ ―CONR₅R₆ ―NHCONR₅R₆
wherein R₄ is cyano, amino, hydroxy or C₁-C₄ alkoxy; R₅, R₆ and R₇, the same or different, are hydrogen or C₁-C₄ alkyl.; or a pharmaceutically acceptable salt thereof; and
- a protein kinase inhibitor.

2. A pharmaceutical composition according to claim 1 wherein the protein kinase inhibitor is selected from STI571, ZD-1839, OSI-774, PKI 166, EKB-569, GW572016, CEP 2563, UCN-01, GCP 41251 (STI 412), Safingol, Perifosine, SU 5416, CGP 79787, CP-564959, ZD 6474, ZD 2171, SU-11248, Flavopiridol, and CI-202.

3. A pharmaceutical composition according to claim 2 wherein the protein kinase inhibitor is selected from STI571, ZD-1839, OSI-774 and SU 5416.

4. A pharmaceutical composition according to claim 1 comprising an acryloyl distamycin derivative of formula (I) wherein R₁ and R₂ are as defined in claim 4, r is 0, m is 0 or 1, n is 4, X and Y are both CH groups and B is selected from: 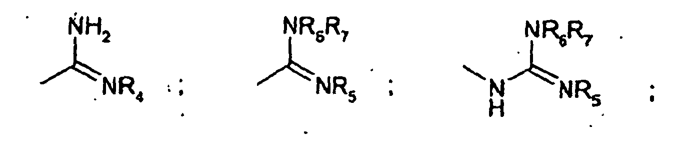
- CN : - CONR₅R₆ : -NHCONR₅R₆
wherein R₄ is cyano or hydroxy and R₅, R₆ and R₇, the same or different, are hydrogen or C₁-C₄ alkyl.

5. A pharmaceutical composition according to claim 4 comprising an acryloyl distamycin derivative of formula (I) wherein R₁ is bromine, R₂ is a group of formula (II) wherein r and m are 0, n is 4, X and Y are CH, B is a group of formula 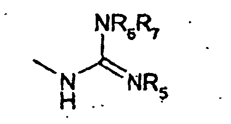 wherein R₅, R₆ and R₇ are hydrogen atoms, optionally in the form of a pharmaceutically acceptable salt.

6. A pharmaceutical composition according to claim 1 comprising an acryloyl distamycin derivative, optionally in the form of a pharmaceutically acceptable salt, selected from
1. N-[5-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino]-1-methyl-1H-pyrrol-2-yl[carbonyl]amino]-1-methyl-1H-pyrrole-2-carboxamide hydrochloride (Brostallicin);
2. N-(5-{[(5-{[(5-{[(2-{[amino(imino)methyl]amino}propyl)amino]carbonyl}-1-methyl-1H-pyrrol-3-yl)amino]carbonyl}-1-metthyl-1H-pyrrol-3-yl)amino]carbonyl}-1-methyl-1H-Pyrrol-3-yl)-4-[(2-bromoacryloyl)amino]-1-methyl-1H-pyrrole-2-carboxamide hydrochloride;
3. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl}amino]carbonyl}-1-methyl-1H-pyrrol-3-yl)-4-[(2-bromoacryloyl)amino]-1-methyl-1H-pyrrole-2-carboxamide hydrochloride;
4. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-imidazole-2-carboxamide hydrochloride;
5. N-(5-{[(5-{[(5-{[(3-amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromoacryloyl)amino]-1-methyl-1H-pyrazole-5-carboxamide hydrochloride;
6. N-(5-{[(5-{[(5-{[(3-amino-3-oxopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromoacryloyl)amino]-1-methyl-1H-pyrazole-5-carboxamide;
7. N-(5-{[(5-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-chloroacryloyl)amino]-1-methyl-1H-pyrrole-2-carboxamide hydrochloride;
8. N-(5-{[(5-{[(3-{[amino(imino)methyl]amino}propyl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride;
9. N-(5-{[(5-{[(3-amino-3-iminopropyl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride; and
10. N-{5-[({5-[({5-[({3-[(aminocarbonyl)amino]propyl}amino)carbonyl]-1-methyl-1H-pyrrol-3-yl}amino)carbonyl]-1-methyl-1H-pyrrol-3-yl}amino)carbonyl]-1-methyl-1H-pyrrol-3-yl}-4-[(2-bromoacryloyl)amino]-1-methyl-1H-pyrrole-2-carboxamide.

7. A pharmaceutical composition comprising a pharmaceutically acceptable carrier or excipient and, as active ingredient,
- N-[5-[[[5-[[[2-[(aminoiminomethyl)amino]ethyl)amino]carbonyl]-1-methyl-1H-pyrrol-3-yl]amino]carbonyl]-1-methyl-1H-pyrrol-3-yl]-4-[[{4-[(2-bromo-1-oxo-2-propenyl)amino]-1-methyl-1H-pyrrol-2-yl[carbonyl]amino]-1-methyl-1H-pyrrole-2-carboxamide hydrochloride (Brostallicin); and
- a protein kinase inhibitor selected from STI571, ZD-1839, OSI-774, and SU 5416.

8. Products comprising an acryloyl distamycin derivative of formula (I): 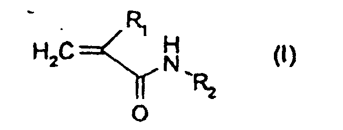 wherein:
R₁ is a bromine or chlorine atom;
R₂ is a distamycin or is a group of formula (II) 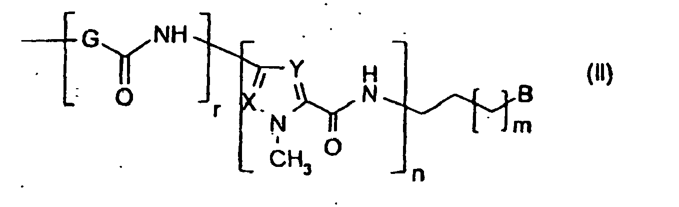 wherein
m is an integer from 0 to 2;
n is an integer from 2 to 5;
r is 0 or 1;
X and Y are, the same or different and independently for each heterocyclic ring, a nitrogen atom or a CH group;
G is phenylene, a 5 or 6 membered saturated or unsaturated heterocyclic ring with from 1 to 3 heteroatoms selected among N, O or S, or it is a group of formula (III) below: 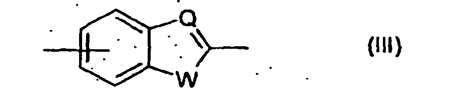 wherein Q is a nitrogen atom or a CH group and W is an oxygen or sulfur atom or it is a group NR₃ wherein R₃ is hydrogen or C₁-C₄ alkyl;
B is selected from the group consisting of 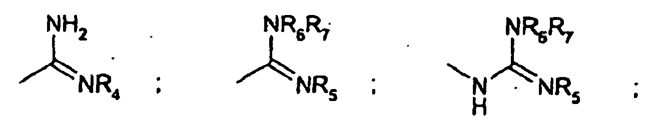 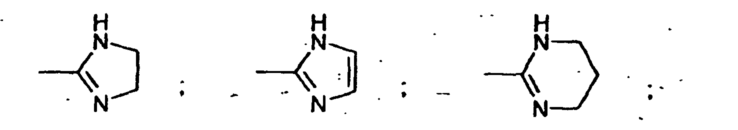
―CN ―NR₅R₆ ―CONR₅R₆ ―NHCONR₅R₆
wherein R₄ is cyano, amino, hydroxy or C₁-C₄ alkoxy; R₅, R₆ and R₇, the same or different, are hydrogen or C₁-C₄ alkyl.; or a pharmaceutically acceptable salt thereof; and a protein kinase inhibitor, as a combined preparation for simultaneous, separate or sequential use in the treatment of tumors.

9. Products according to claim 8 wherein the protein kinase inhibitor is selected from STI571, ZD-1839, OSI-774, PKI 166, EKB-569, GW572016, CEP 2563, UCN-01, GCP 41251 (STI 412), Safingol, Perifosine, SU 5416, CGP 79787, CP-564959, ZD 6474, ZD 2171, SU-11248, Flavopiridol, and CI-202.

10. Products according to claim 9 wherein the protein kinase inhibitor is selected from STI571, ZD-1839, OSI-774 and SU 5416.

11. Products according to claim 8 comprising an acryloyl distamycin derivative of formula (I) wherein R₁ is bromine, R₂ is a group of formula (II) wherein r and m are 0, n is 4, X and Y are CH, B is a group of formula 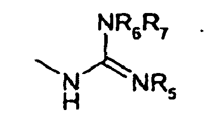 wherein R₅, R₆ and R₇ are hydrogen atoms, optionally in the form of a pharmaceutically acceptable salt.

12. Products according to claim 8 wherein the acryloyl distamycin derivative is selected from the group as defined in claim 6.

13. Products comprising the acryloyl distamycin derivative N-[5-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino]-1-methyl-1H-pyrrol 2-yl[carbonyl]amino]-1-methyl-1H-pyrrole-2-carboxamide hydrochloride (Brostallicin), and a protein kinase inhibitor selected from STI571, ZD-1839, OSI-774, and SU 5416; as a combined preparation for simultaneous, separate or sequential use in the treatment of tumors.

14. Use of an acryloyl distamycin derivative of formula (I), as defined in claim 1, in the preparation of a medicament to be used in combination therapy with a protein kinase inhibitor, in the treatment of tumors.

15. Use according to claim 14 wherein the medicament further comprises the said protein kinase inhibitor.

16. Use according to claims 14 or 15 wherein the protein kinase inhibitor is as defined in claim 2.

17. Use according to claims 14 or 15 wherein the acryloyl distamycin derivative is selected from the group as defined in claim 6.

18. Use of the acryloyl distamycin derivative N-[S-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino]-1-methyl-1H-pyrrol-2-yl[carbonyl]amino]-1-methyl-1H-pyrrole-2-carboxamide hydrochloride (Brostallicin), in the preparation of a medicament to be used in combination therapy with a protein kinase inhibitor selected from ST1571, ZD-1839, OSI-774, and SU 5416, in the treatment of tumors.

19. Use according to any one of claims from 11 to 18 wherein the tumor is selected from breast, ovary, lung, colon, kidney, stomach, pancreas, liver, melanoma, leukemia and brain tumors.

20. Use of an acryloyl distamycin derivative of formula (I), as defined in claim 1, in the preparation of a medicament to be used in combination therapy with a protein kinase inhibitor, in the prevention or treatment of metastasis or in the treatment of tumors by inhibition ofangiogenesis.

21. Use according to claim 20 wherein the medicament further comprises the said protein kinase inhibitor.

22. Use of the acryloyl distamycin derivative of formula (I) as defined in claim 1, and of a protein kinase inhibitor, for manufacturing a medicament for treating a mammal, including humans, suffering from a neoplastic disease state.

23. A use according to claim 22 wherein the acryloyl distamycin derivative is N-[5-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino)-1-methyl-1H-pyrrol-2-yl[carbonyl]amino]-1-methyl-1H-pyrrole-2-carboxamide hydrochloride (Brostallicin), and .the protein kinase inhibitor is selected from STI571, ZD-1839, OSI-774, and SU 5416.

24. Use of a combined preparation comprising a protein kinase inhibitor and an acryloyl distamycin derivative of formula (I), as defined in claim 1, for manufacturing a medicament for lowering the side effects caused by antineoplastic therapy with an antineoplastic agent, in a mammal in need thereof including humans.

25. A use according to claim 24 wherein the acryloyl distamycin derivative is N-[5-[[[5-[[[2-[(aminoiminomethyl)amino]ethyl]amino]carbonyl]-1-methyl-1H-pyrrol-3-yl]amino]carbonyl]-1-methyl- 1H-pyrrol-3-yl]-4-[[[4-[(2-bmmo-1-oxo-2-propenyl)amino]-1-méthyl-1H-pyrrol-2-yl[carbonyl]amino]-1-metyl-1H-pyrrole-2-carboxamide hydrochloride (Brostallicin), and the protein kinase inhibitor is selected from STI571, ZD-1839, OSI-774, and SU 5416.

## Patentansprüche

1. Eine pharmazeutische Zusammensetzung, die folgendes umfasst: einen pharmazeutisch verträglichen Träger oder Bindemittel umfaßt und, als einen aktiven Bestandteil,
ein Acryloyldistamycinderivat der Formel (I): 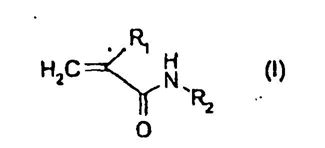 worin:
R₁ ein Brom- oder Chloratom ist;
R₂ ist ein Distamycin oder eine Gruppe der Formel (II) 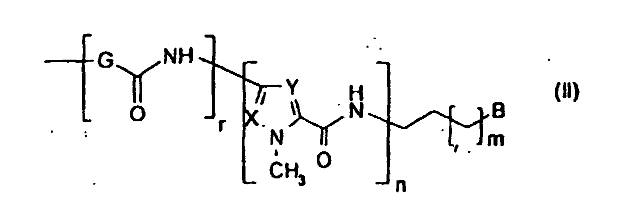
worin
m eine ganze Zahl von 0 bis 2 ist,
n ist eine ganze Zahl von 2 bis 5;
r ist 0 oder 1;
X und Y sind, gleich oder unterschiedlich und unabhängig für jeden heterocyclischen Ring, ein Stickstoffatom oder eine CH Gruppe;
G ist Phenylen, ein 5- oder 6-gliedriger gesättigter oder ungesättigter heterocyclischer Ring mit von 1 bis 3 Heteroatomen gewählt aus N, O oder S, oder es ist eine Gruppe der Formel (III) unten: 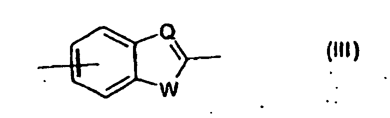 worin Q ein Stickstoffatom oder eine CH Gruppe ist, und W ist ein Sauerstoff- oder Schwefelatom, oder es ist eine Gruppe NR₃, worin R₃ Wasserstoff oder C₁-C₄ Alkyl ist; B ist gewählt aus der Gruppe bestehend aus 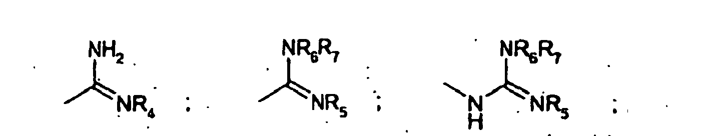 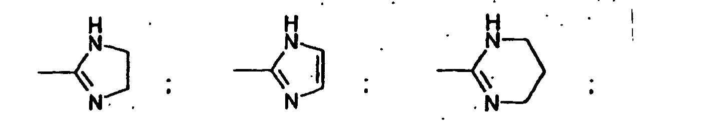
―CN ; ―NR₅R₆- ; ―NHCONR₅R₆ ; ―NHCONR₅R₆
worin R₄ Cyano, Amino, Hydroxy oder C₁-C₄ Alkoxy ist; R₅, R₆ und R₇ sind, gleich oder unterschiedlich, Wasserstoff oder C₁-C₄ Alkyl; oder ein pharmazeutisch verträgliches Salz davon; und
einen Proteinkinaseinhibitor.

2. Eine pharmazeutische Zusammensetzung gemäß Anspruch 1, worin der Proteinkinaseinhibitor gewählt ist aus STI571, ZD-1839, OSI-774, PKI 166, EKB-569, GW572016, CEP 2563, UCN-01, GCP 41251 (STI 412), Safingol, Perifosin, SU 5416, CGP 79787, CP-564959, ZD 6474, ZD 2171, SU-11248, Flavopiridol und CI-202.

3. Eine pharmazeutische Zusammensetzung gemäß Anspruch 2, worin der Proteinkinaseinhibitor gewählt ist aus STI571, ZD-1839, OSI-774 und SU 5416.

4. Eine pharmazeutische Zusammensetzung gemäß Anspruch 1, umfassend ein Acryloyldistamycinderivat der Formel (I), worin R₁ und R₂ wie in Anspruch 4 definiert sind, r ist 0, m ist 0 oder 1, n ist 4, X und Y sind beide CH Gruppen und B ist gewählt aus: 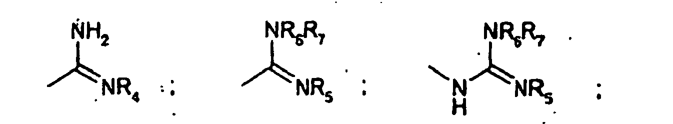 ―CN; ―CONR₅R₆; ―NHCONR₅R₆
worin R₄ Cyano oder Hydroxy ist und R₅, R₆ und R₇ sind, gleich oder unterschiedlich, Wasserstoff oder C₁-C₄ Alkyl.

5. Eine pharmazeutische Zusammensetzung gemäß Anspruch 4, umfassend ein Acryloyldistamycinderivat der Formel (I), worin R₁ Brom ist, R₂ ist eine Gruppe der Formel (II), worin r und m 0 sind, n ist 4, X und Y sind CH, B ist eine Gruppe der Formel 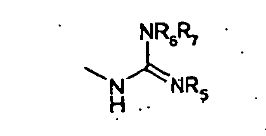 worin R₅, R₆ und R₇ Wasserstoffatome sind, wahlweise in der Form eines pharmazeutisch verträglichen Salzes.

6. Eine pharmazeutische Zusammensetzung gemäß Anspruch 1, umfassend ein Acryloyldistamycinderivat, wahlweise in der Form eines pharmazeutisch verträglichen Salzes, gewählt aus
1. N-[5-[[[5-[[[2-[(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl[carbonyl]amino]-1-methyl-1H-pyrrol-2-carboxamidhydrochlorid (Brostallicin);
2. N-(5-{[(5-{[(5-{[(2-{[Amino(imino)methyl]amino} propyl)amino]carbonyl}-1-methyl-1H-pyrrol-3-yl)amino]carbonyl} -1-methyl-1H-pyrrol-3-yl)amino]carbonyl}-1-methyl-1H-pyrrol-3-yl)-4-[(2-bromacryloyl)amino]-1-methyl-1H-pyrrol-2-carboxamidhydrochlorid;
3. N-(5-{[(5-{[(5-{[(3-Amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromacryloyl)amino]-1-methyl-1H-pyrrol-2-carboxamidhydrochlorid;
4. N-(5-{[(5-{[(5-{[(3-Amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromacryloyl)amino]-1-methyl-1H-imidazol-2-carboxamidhydrochlorid;
5. N-(5-{[(5-{[(5-{[(3-Amino-3-iminopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromacryloyl)amino]-1-methyl-1H-pyrazol-5-carboxamidhydrochlorid;
6. N-(5-{[(5-{[(5-{[(3-Amino-3-oxopropyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-3-[(2-bromacryloyl)amino]-1-methyl-1H-pyrazol-5-carboxamid;
7. N-(5-{[(5-{[(5-{[(2-{[Amino(imino)methyl]amino} ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-chloracryloyl)amino]-1-methyl-1H-pyrrol-2-carboxamidhydrochlorid;
8. N-(5-{[(5-{[(3-{[Amino(imino)methyl]amino} propyl)amino]carbonyl}-1-methyl-1H-pyrrol-3-yl)amino]carbonyl} -1-methyl-1H-pyrrol-3-yl)-4- [(2-bromacryloyl) amino] -1-methyl-1H-pyrrol-2-carboxamidhydrochlorid;
9. N-(5-{[(5-{[(3-Amino-3-iminopropyl)amino]carbonyl}-1-methyl-1H-pyrrol-3-yl)amino]carbonyl}-1-methyl-1H-pyrrol-3-yl)-4-[(2-bromacryloyl)amino]-1-methyl-1H-pyrrol-2-carboxamidhydrochlorid; und
10. N-{5-[({5-[({5-[({3-[(Aminocarbonyl)amino]propyl} amino)carbonyl]-1-methyl-1H-pyrrol-3-yl}amino)carbonyl}-1-methyl-1H-pyrrol-3-yl}amino)carbonyl]-1-methyl-1H-pyrrol-3-yl} -4-[(2-bromacryloyl)amino]-1-methyl-1H-pyrrol-2-carboxamid.

7. Eine pharmazeutische Zusammensetzung, die folgendes umfasst: einen pharmazeutisch verträglichen Träger oder Bindemittel und, als einen aktiven Bestandteil, N-[5-[[[5-[[[2-[(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl(carbonyl]amino]-1-methyl-1H-pyrrol-2-carboxamidhydrochlorid (Brostallicin); und
- ein Proteinkinaseinhibitor gewählt aus STI571, ZD-1839, OSI-774 und SU 5416.

8. Produkte, umfassend ein Acryloyldistamycinderivat der Formel (I) : 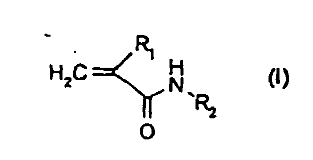 worin:
R₁ ein Brom- oder Chloratom ist;
R₂ ist ein Distamycin oder eine Gruppe der Formel (II) 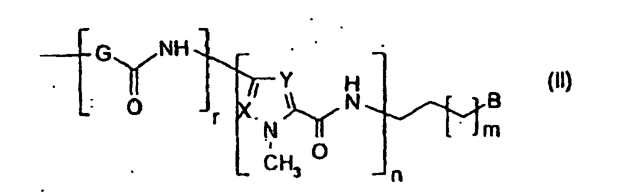
worin
m eine ganze Zahl von 0 bis 2 ist,
n ist eine ganze Zahl von 2 bis 5;
r ist 0 oder 1;
X und Y sind, gleich oder unterschiedlich, und unabhängig für jeden heterocyclischen Ring, ein Stickstoffatom oder eine CH Gruppe;
G ist Phenylen, ein 5- oder 6-gliedriger gesättigter oder ungesättigter heterocyclischer Ring mit von 1 bis 3 Heteroatomen gewählt aus N, O oder S, oder es ist eine Gruppe der Formel (III) unten: 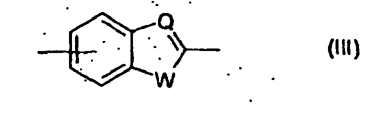 worin Q ein Stickstoffatom oder eine CH Gruppe ist und W ist ein Sauerstoff- oder Schwefelatom, oder es ist eine Gruppe NR₃, worin R₃ Wasserstoff oder C₁-C₄ Alkyl ist; B ist gewählt aus der Gruppe bestehend aus 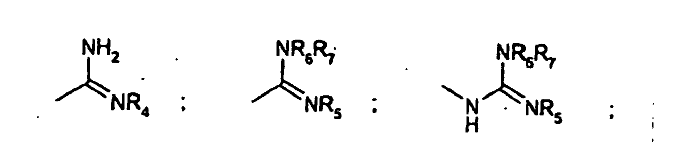 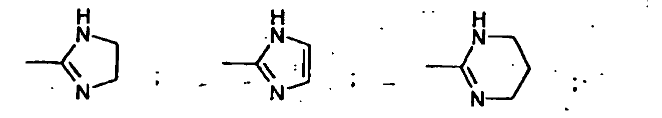
―CN; ―NR₅R₆; ―CONR₅R₆; ―NHCONR₅R₆
worin R₄ Cyano, Amino, Hydroxy oder C₁-C₄ Alkoxy ist; R₅, R₆ und R₇ sind, gleich oder unterschiedlich, Wasserstoff oder C₁-C₄ Alkyl oder ein pharmazeutisch verträgliches Salz davon; und einen Proteinkinaseinhibitor, als eine kombinierte Zubereitung für die geleichzeitige, getrennte oder aufeinanderfolgende Verwendung in der Behandlung von Tumoren.

9. Produkte gemäß Anspruch 8, worin der Proteinkinaseinhibitor gewählt ist aus STI571, ZD-1839, OSI-774, PKI 166, EKB-569, GW572016, CEP 2563, UCN-01, GCP 41251 (STI 412), Safingol, Perifosin, SU 5416, CGP 79787, CP-564959, ZD 6474, ZD 2171, SU-11248, Flavopiridol und CI-202.

10. Produkte gemäß Anspruch 9, worin der Proteinkinaseinhibitor gewählt ist aus STI571, ZD-1839, OSI-774 und SU 5416.

11. Produkte gemäß Anspruch 8, umfassend ein Acryloyldistamycinderivat der Formel (I), worin R₁ Brom ist, R₂ ist eine Gruppe der Formel (II), worin r und m 0 sind, n ist 4, X und Y sind CH, B ist eine Gruppe der Formel 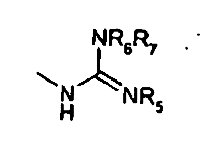 worin R₅, R₆ und R₇ Wasserstoffatome sind, wahlweise in der Form eines pharmazeutisch verträglichen Salzes.

12. Produkte gemäß Anspruch 8, worin das Acryloyldistamycinderivat gewählt ist aus der Gruppe wie in Anspruch 6 definiert.

13. Produkte, umfassend das Acryloyldistamycinderivat N-[5-[[[5-[[[2-[(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl[carbonyl]amino]-1-methyl-1H-pyrrol-2-carboxamidhydrochlorid (Brostallicin) und einen Proteinkinaseinhibitor gewählt aus STI571, ZD-1839, OSI-774 und SU 5416; als eine kombinierte Zubereitung für die gleichzeitige, getrennte oder aufeinanderfolgende Verwendung in der Behandlung von Tumoren.

14. Verwendung eines Acryloyldistamycinderivats der Formel (I), wie in Anspruch 1 definiert, in der Herstellung eines Medikaments für die Verwendung in der Kombinationstherapie mit einem Proteinkinaseinhibitor in der Behandlung von Tumoren.

15. Verwendung gemäß Anspruch 14, worin das Medikament weiter den Proteinkinaseinhibitor umfaßt.

16. Verwendung gemäß den Ansprüchen 14 oder 15, worin der Proteinkinaseinhibitor wie in Anspruch 2 definiert ist.

17. Verwendung gemäß den Ansprüchen 14 oder 15, worin das Acryloyldistamycinderivat gewählt ist aus der Gruppe wie in Anspruch 6 definiert.

18. Verwendung des Acryloyldistamycinderivats N-[5-[[[5-[[[2-[(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl[carbonyl]amino]-1-methyl-1H-pyrrol-2-carboxamidhydrochlorid (Brostallicin) in der Herstellung eines Medikaments für die Verwendung in der Kombinationstherapie mit einem Proteinkinaseinhibitor gewählt aus STI571, ZD-1839, OSI-774 und SU 5416 in der Behandlung von Tumoren.

19. Verwendung gemäß irgendeinem der Ansprüche 11 bis 18, worin der Tumor gewählt ist aus Brust-, Eierstock-, Lungen-, Dickdarm- , Nieren-, Magen-, Bauchspeicheldrüsen-, Leber-, Melanom, Leukämie und Gehirntumoren.

20. Verwendung eines Acryloyldistamycinderivats der Formel (I), wie in Anspruch 1 definiert, in der Herstellung eines Medikaments für die Verwendung in der Kombinationstherapie mit einem Proteinkinaseinhibitor in der Vorbeugung oder Behandlung von Metastasen oder in der Behandlung von Tumoren durch Hemmung der Angiogenese.

21. Verwendung gemäß Anspruch 20, worin das Medikament weiter den Proteinkinaseinhibitor umfaßt.

22. Verwendung des Acryloyldistamycinderivats der Formel (I), wie in Anspruch 1 definiert, und eines Proteinkinaseinhibitors zur Herstellung eines Medikaments für die Behandlung eines Säugetiers, einschließlich Menschen, das an einem neoplastischen Erkrankungszustand leidet.

23. Eine Verwendung gemäß Anspruch 22, worin das Acryloyldistamycinderivat N-[5-[[[5-[[[2-[(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl[carbonyl]amino)-1-methyl-1H-pyrrol-2-carboxamidhydrochlorid (Brostallicin) ist und der Proteinkinaseinhibitor gewählt ist aus STI571, ZD-1839, OSI-774 und SU 5416.

24. Verwendung einer kombinierten Herstellung, umfassend einen Proteinkinaseinhibitor und ein Acryloyldistamycinderivat der Formel (I), wie in Anspruch 1 definiert, für die Herstellung eines Medikaments zur Abschwächung der Nebenwirkungen, die durch antineoplastische Therapie mit einem antineoplastischen Wirkstoff verursacht sind, in einem Säugetier, das es benötigt, einschließlich Menschen.

25. Eine Verwendung gemäß Anspruch 24, worin das Acryloyldistamycinderivat N-[5-[[[5-[[[2-[(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl[carbonyl]amino]-1-methyl-1H-pyrrol-2-carboxamidhydrochlorid (Brostallicin) ist, und der Proteinkinaseinhibitor gewählt ist aus STI571, ZD-1839, OSI-774 und SU 5416.

## Revendications

1. Composition pharmaceutique comprenant un support ou excipient pharmaceutiquement tolérable et, comme ingrédient actif,
- un dérivé de distamycine acryloyle de la formule (I): 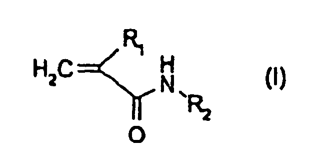 dans laquelle:
R₁ est un atome de brome ou de chlore;
R₂ est un distamycine ou un groupe de la formule (II) 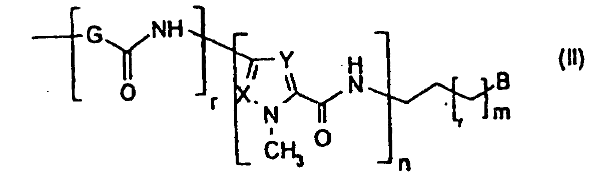 dans laquelle
m est un nombre entier de 0 à 2;
n est un nombre entier de 2 à 5;
r est 0 ou 1;
X et Y sont, les mêmes ou différents et indépendamment de chaque anneau hétérocyclique, un atome d'azote ou un groupe CH;
G est phénylène, un anneau hétérocyclique saturé ou insaturé à 5 ou 6 membres avec de 1 à 3 hétéroatomes choisis parmi N, O ou S, ou est un groupe de la formule (III) ci-dessous: 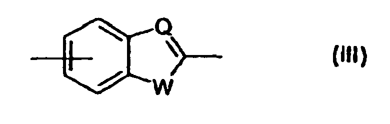 dans laquelle Q est un atome d'azote ou un groupe CH et W est un atome de soufre ou d'oxygène ou est un groupe NR₃ dans lequel R₃ est hydrogène ou un alkoyle C₁-C₄;
B est choisi parmi le groupe constitué de 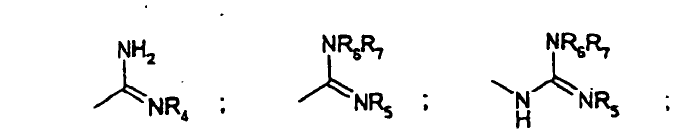 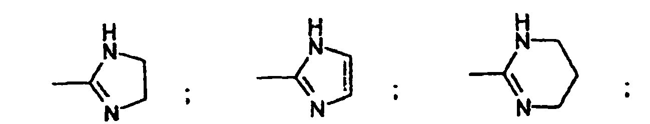
―CN; ―NR₅R₆-; ―CONR₅R₆; ―NHCONR₅R₆
dans lequel R₄ est cyano, amino, hydroxy ou alkoxy C₁-C₄; R₅, R₆ et R₇, les mêmes ou différents, sont hydrogène ou un alkoyle C₁-C₄;
ou un sel de distamycine pharmaceutiquement tolérable; et
- un inhibiteur de protéine kynase.

2. Composition pharmaceutique selon la revendication 1 dans laquelle l'inhibiteur de protéine kynase est choisi parmi STI571, ZD-1839, OSI-774, PKI 166, EKB-569, GW572016, CEP 2563, UCN-01, GCP 41251 (STI 412), Safingol, Perifosine, SU 5416, CGP 79787, CP-564959, ZD 6474, ZD 2171, SU-11248, Flavopiridol, et CI-202.

3. Composition pharmaceutique selon la revendication 2 dans laquelle l'inhibiteur de protéine kynase est choisi parmi STI571, ZD-1839, OSI-774 et SU 5416.

4. Composition pharmaceutique selon la revendication 1 comprenant un dérivé de distamycine acryloyle de formule (I) dans laquelle R₁ et R₂ sont tels que définis dans la revendication 4, r est 0, m est 0 ou 1, n est 4, X et Y sont tous les deux des groupes CH et B est choisi à partir: 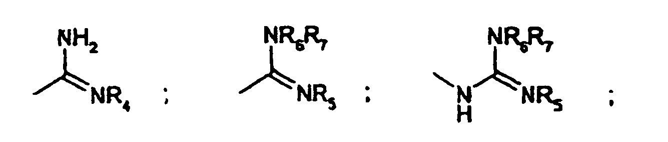
―CN ; ―CONR₅R₆ ; ―NHCONR₅R₆
dans lequel R₄ est cyano ou hydroxy et R₅, R₆ et R₇, les mêmes ou différents, sont hydrogène
ou un alkoyle C₁-C₄.

5. Composition pharmaceutique selon la revendication 1 comprenant un dérivé de distamycine acryloyle de formule (I) dans laquelle R₁ est brome, R₂ est un groupe de formule (II) dans laquelle r et m sont 0, n est 4, X et Y sont CH, B est un groupe de formule 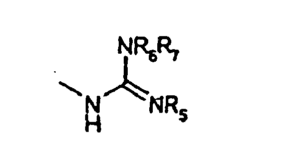 dans laquelle R₅, R₆ et R₇ sont des atomes d'hydrogène, de façon optionnelle sous la forme d'un sel pharmaceutiquement tolérable.

6. Composition pharmaceutique selon la revendication 1 comprenant un dérivé de distamycine acryloyle, de façon optionnelle sous la forme d'un sel pharmaceutiquement tolérable, choisi à partir
1. N-[5-[[[5-[[[2-[(aminoiminométhyle)amino]éthyle]amino]carbonyle]-1-méthyle-1H-pyrrole-3-yl]amino]carbonyle]-1-méthyle-1H-pyrrole-3-yl]-4-[[[4-[(2-bromo-1-oxo-2-propényle)amino]-1-méthyle-1H-pyrrole-2-yl[carbonyle]amino]-1-méthyle-1H-pyrrole-2-carboxamide chlorhydrate (Brostallicine);
2. N-(5-{[(5-{[(5-{[(2-{[amino(imino)méthyle]amino}propyle)amino]carbonyle}-1-méthyle-1H-pyrrole-3-yl)amino]carbonyle}-1-méthyle-1H-pyrrole-3-yl)amino]carbonyle}-1-méthyle-1H-pyrrole-3-yl)-4-[(2-bromoacryloyle)amino]-1-méthyle-1H-pyrrole-2-carboxamide chlorhydrate;
3. N-(5- {[(5-{[(5-{[(3-amino-3-iminopropyle)amino]carbonyle}-1-méthyle-1H-pyrrole-3-yl) amino]carbonyle}-1-méthyle-1H-pyrrole-3-yl)amino]carbonyle}-1-méthyle-1H-pyrrole-3-yl)-4-[(2-bromoacryloyle)amino]-1-méthyle-1 H-pyrrole-2-carboxamide chlorhydrate;
4. N-(5-{[(5-{[(5-{[(3-amino-3-iminopropyle)amino]carbonyle}-1-méthyle-1H-pyrrole-3-yl) amino]carbonyle}-1-méthyle-1H-pyrrole-3-yl)amino]carbonyle}-1-méthyle-1H-pyrrole-3-yl)-4-[(2-bromoacryloyle)amino]-1-méthyle-1H-imidazole-2-carboxamide chlorhydrate;
5. N-(5-{[(5-{[(5-{[(3-amino-3-iminopropyle)amino]carbonyle}-1-méthyle-1H-pyrrole-3-yl) amino]carbonyle}-1-méthyle-1H-pyrrole-3-yl)amino]carbonyle}-1-méthyle-1H-pyrrole-3-yl)-3-[(2-bromoacryloyle)amino]-1-méthyle-1H-pyrazole-5-carboxamide chlorhydrate;
6. N-(5-{[(5-{[(5-{[(3-amino-3-oxopropyle)amino]carbonyle}-1-méthyle-1H-pyrrole-3-yl) amino]carbonyle}-1-méthyle-1H-pyrrole-3-yl)amino]carbonyle}-1-méthyle-1H-pyrrole-3-yl)-3-[(2-bromoacryloyle)amino]-1-méthyle-1H-pyrazole-5-carboxamide;
7. N-(5-{[(5-{[(5-{[(2-{[amino(imino)méthyle]amino}éthyle)amino]carbonyle}-1-méthyle-1H-pyrrole-3-yl)amino]carbonyle}-1-méthyle-1H-pyrrole-3-yl)amino]carbonyle}-1-méthyle-1H-pyrrole-3-yl)-4-[(2-chloroacryloyle)amino]-1-méthyle-1H-pyrrole-2-carboxamide chlorhydrate;
8. N-(5-{[(5-{[(3-{[amino(imino)méthyle]amino}propyle)amino]carbonyle}-1-méthyle-1H-pyrrole-3-yl)amino]carbonyle} -1-méthyle-1 H-pyrrole-3-yl)-4-[(2-bromoacryloyle)amino]-1 - méthyle-1H-pyrrole-2-carboxamide chlorhydrate;
9. N-(5-{[(5-{[(3-amino-3-iminopropyle)amino]carbonyle}-1-méthyle-1H-pyrrole-3-yl) amino]carbonyle}-1-méthyle-1H-pyrrole-3-yl)-4-[(2-bromoacryloyle)amino]-1-méthyle-1H-pyrrole-2-carboxamide chlorhydrate; et
10. N-{5-[({5-[({5-[({3-[(aminocarbonyle)amino]propyle}amino)carbonyle]-1-méthyle-1H-pyrrole-3-yl}amino)carbonyle]-1-méthyle-1H-pyrrole-3-yl}amino)carbonyle]-1-méthyle-1H-pyrrole-3-yl}-4-[(2-bromoacryloyle)amino]-1-méthyle-1H-pyrrole-2-carboxamide.

7. Composition pharmaceutique comprenant un support ou excipient pharmaceutiquement tolérable et, comme ingrédient actif,
- N-[5-[[[5-[[[2-[(aminoiminométhyle)amino]éthyle]amino]carbonyle]-1-méthyle-1H-pyrrole-3-yl]amino]carbonyle]-1-méthyle-1H-pyrrole-3-yl]-4-[[[4-[(2-bromo-1-oxo-2-propényle)amino]-1-méthyle-1H-pyrrole-2-yl[carbonyle]amino]-1-méthyle-1H-pyrrole-2-carboxamide chlorhydrate (Brostallicine); et
- un inhibiteur de protéine kynase choisi parmi STI571, ZD-1839, OSI-774, et SU 5416.

8. Produits comprenant un dérivé de distamycine acryloyle de la formule (I): 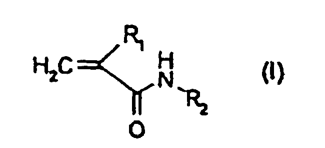 dans laquelle:
R₁ est un atome de brome ou de chlore;
R₂ est un distamycine ou un groupe de la formule (II) 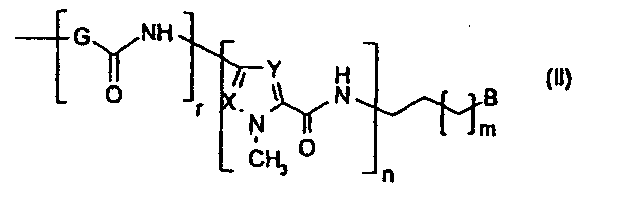
dans laquelle
m est un nombre entier de 0 à 2;
n est un nombre entier de 2 à 5;
r est 0 ou 1 ;
X et Y sont, les mêmes ou différents et indépendamment de chaque anneau hétérocyclique, un atome d'azote ou un groupe CH;
G est phénylène, un anneau hétérocyclique saturé ou insaturé à 5 ou 6 membres avec de 1 à 3 hétéroatomes choisis parmi N, O ou S, ou est un groupe de la formule (III) ci-dessous: 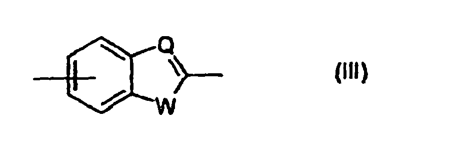 dans laquelle Q est un atome d'azote ou un groupe CH et W est un atome de soufre ou d'oxygène ou est un groupe NR₃ dans lequel R₃ est hydrogène ou un alkoyle C₁-C₄;
B est choisi parmi le groupe constitué de 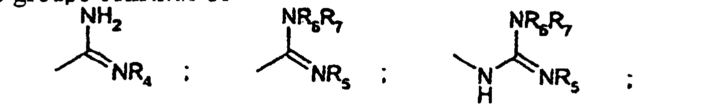 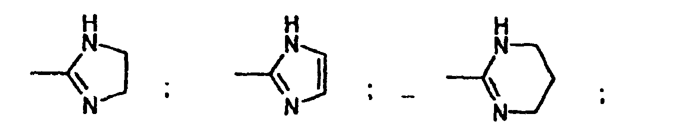
- CN ; -NR₅R₆; - CONR₅R_{6;} - NHCONR₅R₆
dans lequel R₄ est cyano, amino, hydroxy ou alkoxy C₁-C₄; R₅, R₆ et R₇, les mêmes ou différents, sont hydrogène ou un alkoyle C₁-C₄;
ou un sel de distamycine pharmaceutiquement tolérable; et un inhibiteur de protéine kynase, en tant que préparation combinée pour une utilisation simultanée, séparée ou séquentielle dans le traitement des tumeurs.

9. Produits selon la revendication 8 dans lesquels l'inhibiteur de protéine kynase est choisi parmi STI571, ZD-1839, OSI-774, PKI 166, EKB-569, GW572016, CEP 2563, UCN-01, GCP 41251 (STI 412), Safingol, Perifosine, SU 5416, CGP 79787, CP-564959, ZD 6474, ZD 2171, SU-11248, Flavopiridol, et CI-202.

10. Produits selon la revendication 9 dans lesquels l'inhibiteur de protéine kynase est choisi parmi STI571, ZD-1839, OSI-774 et SU 5416.

11. Produits selon la revendication 8 comprenant un dérivé de distamycine acryloyle de formule (I) dans laquelle R₁ est brome, R₂ est un groupe de formule (II) dans laquelle r et m sont 0, n est 4, X et Y sont CH, B est un groupe de formule 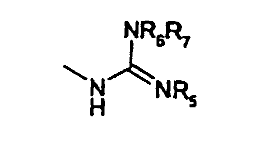 dans laquelle R₅, R₆ et R₇ sont des atomes d'hydrogène, de façon optionnelle sous la forme d'un sel pharmaceutiquement tolérable.

12. Produits selon la revendication 8 dans lesquels le dérivé de distamycine acryloyle est choisi parmi le groupe tel que défini dans la revendication 6.

13. Produits comprenant le dérivé de distamycine acryloyle N-[5-[[[5-[[[2-[(aminoiminométhyle)amino]éthyle]amino]carbonyle]-1-méthyle-1H-pyrrole-3-yl]amino] carbonyle]-1-méthyle-1H-pyrrole-3-yl]-4-[[[4-[(2-bromo-1-oxo-2-propényle)amino]-1-méthyle-1H-pyrrole-2-yl[carbonyle]amino]-1-méthyle-1H-pyrrole-2-carboxamide chlorhydrate (Brostallicine), et un inhibiteur de protéine kynase choisi parmi STI571, ZD-1839, OSI-774, et SU 5416; en tant que préparation combinée pour une utilisation simultanée, séparée ou séquentielle dans le traitement des tumeurs.

14. Utilisation d'un dérivé de distamycine acryloyle de la formule (I), tel que défini dans la revendication 1 dans la préparation d'un médicament pour l'utilisation dans une thérapie en combinaison avec un inhibiteur de protéine kynase, dans le traitement de tumeurs.

15. Utilisation selon la revendication 14 dans laquelle le médicament comprend en outre ledit inhibiteur de protéine kynase.

16. Utilisation selon les revendications 14 ou 15 dans laquelle l'inhibiteur de protéine kynase est tel que défini dans la revendication 2.

17. Utilisation selon les revendication 14 ou 15 dans laquelle le dérivé de distamycine acryloyle est choisi parmi le groupe tel que défini dans la revendication 6..

18. Utilisation du dérivé de distamycine acryloyle N-[5-[[[5-[[[2-[(aminoiminométhyle) amino]éthyle]amino]carbonyle]-1-méthyle-1H-pyrrole-3-yl]amino]carbonyle]-1-méthyle-1H-pyrrole-3-yl]-4-[[[4-[(2-bromo-1-oxo-2-propényle)amino]-1-méthyle-1H-pyrrole-2-yl [carbonyle]amino]-1-méthyle-1H-pyrrole-2-carboxamide chlorhydrate (Brostallicine), dans la préparation d'un médicament pour l'utilisation dans une thérapie en combinaison avec un inhibiteur de protéine kynase choisi parmi STI1571, ZD-1839, OSI-774, et SU 5416, dans le traitement de tumeurs.

19. Utilisation selon l'une quelconque des revendications 11 à 18 dans laquelle la tumeur est choisie parmi les tumeurs du sein, de l'ovaire, du poumon, du colon, du rein, de l'estomac, du pancréas, du foie, au cerveau, le mélanome et la leucémie.

20. Utilisation d'un dérivé de distamycine acryloyle de la formule (I), tel que défini dans la revendication 1, dans la préparation d'un médicament pour l'utilisation dans une thérapie en combinaison avec un inhibiteur de protéine kynase, dans la prévention ou le traitement de métastases ou dans le traitement de tumeurs par inhibition d'angiogenèse.

21. Utilisation selon la revendication 20 dans laquelle le médicament comprend en outre ledit inhibiteur de protéine kynase.

22. Utilisation d'un dérivé de distamycine acryloyle de la formule (I), tel que défini dans la revendication 1, et d'un inhibiteur de protéine kynase, pour la fabrication d'un médicament pour le traitement d'un mammifère, y compris les humains, souffrant d'un état de maladie néoplastique.

23. Utilisation selon la revendication 22 dans laquelle le dérivé de distamycine acryloyle est N-[5-[[[5-[[[2-[(aminoiminométhyle)amino]éthyle]amino]carbonyle]-1-méthyle-1H-pyrrole-3-yl]amino]carbonyle]-1-méthyle-1H-pyrrole-3-yl]-4-[[[4-[(2-bromo-1-oxo-2-propényle)amino]-1-méthyle-1H-pyrrole-2-yl[carbonyle]amino]-1-méthyle-1H-pyrrole-2-carboxamide chlorhydrate (Brostallicine), et l'inhibiteur de protéine kynase est choisi parmi STI1571, ZD-1839, OSI-774, et SU 5416.

24. Utilisation d'une préparation combinée comprenant un inhibiteur de protéine kynase et un dérivé de distamycine acryloyle de la formule (I), tel que défini dans la revendication 1, pour la fabrication d'un médicament pour diminuer les réactions secondaires causés par une thérapie antinéoplastique avec un agent antinéoplastique, chez les mammifères en ayant besoin, y compris les humains.

25. Utilisation selon la revendication 24 dans laquelle le dérivé de distamycine acryloyle est N-[5-[[[5-[[[2-[(aminoiminométhyle)amino]éthyle]amino]carbonyle]-1-méthyle-1H-pyrrole-3-yl]amino]carbonyle]-1-méthyle-1H-pyrrole-3-yl]-4-[[[4-[(2-bromo-1-oxo-2-propényle)amino]-1-méthyle-1H-pyrrole-2-yl[carbonyle]amino]-1-méthyle-1H-pyrrole-2-carboxamide chlorhydrate (Brostallicine), et l'inhibiteur de protéine kynase est choisi parmi STI1571, ZD-1839, OSI-774, et SU 5416.
